Europäisches Patentamt

(19) **European Patent Office**

**Office européen des brevets**

(11) Numéro de publication: **0 048 675**

**A1**

(12) # DEMANDE DE BREVET EUROPEEN

(21) Numéro de dépôt: **81401470.0**

(22) Date de dépôt: **22.09.81**

(51) Int. Cl.³: **C 02 F 3/28**
**A 01 C 3/02**

(30) Priorité: **24.09.80 FR 8020868**

(43) Date de publication de la demande:
**31.03.82 Bulletin 82/13**

(84) Etats contractants désignés:
**BE CH DE GB IT LI NL SE**

(71) Demandeur: **Société Entreprise Métallurgique d'Armor**
**(S.E.M.A.) S.A.**
**Rue Monge - Zone Industrielle**
**F-22008 Saint-Brieuc(FR)**

(72) Inventeur: **Chasse, Claude**
**22, rue Donizetti**
**F-29200 Brest(FR)**

(72) Inventeur: **Radenac, Pierre**
**52, rue des Villes Moisan**
**F-22440 Ploufragan(FR)**

(74) Mandataire: **Le Guen, Louis François**
**13, rue Emile Bara BP 91**
**F-35800 Dinard Cedex(FR)**

(54) **Appareil de traitement de déchets biochimiques.**

(57) Dans l'appareil les phases d'hydrolyse liquéfactrice, acidogène et méthanogène sont séparées.

Il est constitué par une enceinte (1) allongée et horizontale, contenant un mélange d'effluents déjà traités et de nouveaux déchets en cours de traitement. Les nouveaux déchets sont introduits à une extrémité (2) et les effluents traités sont soutirés à l'autre extrémité (12). L'enceinte (1) est divisée en trois compartiments (27, 28, 29) par deux cloisons séparatrices. Chaque cloison a une ouverture légèrement au dessous du niveau de la surface libre du mélange pour isoler des deux côtés de sa partie basse, en amont, une masse de mélange contenant une population de bactéries en majorité différentes de celle de la masse située en aval de la cloison, et, dans sa partie haute, des volumes de gaz différents. La première cloison sépare le compartiment de la phase d'hydrolyse liquéfactrice (27) de celui de la phase acidogène (28), et la seconde le compartiment de la phase acidogène (28) de celui de la phase méthanogène (29). Dans chaque compartiment, est prévu un lit bactérien régulant le pH.

./..

FIG.1

Appareil de traitement de déchets biochimiques.

La présente invention concerne un appareil de traitement de déchets biochimiques et, plus particulièrement, un appareil de digestion anaérobie de lisiers ou de déchets biologiques analogues.

On sait que le stockage du lisier présente de nombreux inconvénients puisque le rejet du lisier à l'état brut est de plus en plus
discuté en raison de contraintes d'environnement et de fertilisation.
Pour surmonter ces inconvénients, on a proposé de traiter le lisier
soit par fermentation aérobie, soit par fermentation anaérobie. Dans
la présente demande, on ne considérera que la seconde solution qui
entraîne, outre la déodorisation, la production de méthane et qui
confère à l'effluent traité des qualités fertilisantes en ce qui
concerne le taux de matières azotées et la rapidité de l'action après
épandage.

Le déroulement de la fermentation anaérobie se décompose en
trois phases:

    - une phase d'hydrolyse et de liquéfaction, au cours de laquelle des populations de bactéries, spécifiques à cette phase,
      et des enzymes éventuellement surajoutés hydrolysent et liquéfient les matières organiques, en produisant de petites molécules à partir des grosses molécules initiales,

    - une phase acidogène, au cours de laquelle des populations de
      bactéries acidogènes transforment les matières organiques hydrolysées en acides organiques à petites molécules, dont une grande partie est de l'acide acétique, et

- une phase méthanogène, au cours de laquelle des populations de bactéries méthanogènes transforment les acides organiques, l'hydrogène et le gaz carbonique présents dans la solution en méthane.

Les boues résiduaires et le surnageant sont récupérés et, en général, servent d'engrais.

Les appareils les plus connus de fermentation anaérobie de déchets organiques sont généralement constitués par une enceinte close calorifugée et chauffée entre 35 et 37°C, dans laquelle on introduit une charge de déchets organiques, éventullement additionnée d'enzymes et de bactéries adéquats. La fermentation de la charge prend un certain temps. On recueille le mélange de méthane et de gaz carbonique en haut de l'enceinte. Une fois la fermentation terminée, on évacue les boues résiduaires. Ces appareils fonctionnent donc de manière discontinue, ce qui nécessite soit un stockage préalable des déchets avant l'introduction dans l'appareil, soit l'installation de plusieurs appareils à cycles de fonctionnement décalés afin d'obtenir un traitement quasi-continu. Par ailleurs, dans les appareils connus, les deux phases de fermentation acide et de fermentation méthanique coexistent dans le même environnement physique et chimique et le rendement du procédé ainsi que la régulation dépendent de la série des diverses réactions dont la plus lente définit la vitesse de l'ensemble. En pratique, dans ces appareils connus, avec une température de 37° C, il faut compter de l'ordre de 20 jours pour épuiser la digestion dans des conditions économiques.

Pour remédier à la faible efficacité des appareils connus mentionnés ci-dessus, on a proposé de séparer l'une de l'autre les deux phases de fermentation acide et méthanique, de manière à améliorer les conditions de fonctionnement des deux phases séparées et, ainsi, réduire la durée du traitement par rapport au procédé classique. Cette réduction de durée se traduit évidemment par une réduction volumétrique des enceintes de traitement. En fait, dans ces procédés à séparation en deux phases, on sépare les déchets traités, que l'on a soumis à la fermentation acide, en un liquide surnageant renfermant les acides organiques et une boue résiduaire en utilisant des unités de séparation solides-liquides, puis on soumet le liquide surnageant à une fermentation méthanique. La boue résiduaire, après la fermentation méthanique, est en partie recyclée en phase de fermentation acide et le reste est évacué hors de l'appareil de traitement.

Un objet de la présente invention consiste à prévoir un appareil de digestion anaérobie de déchets biologiques dans lequel les phases d'hydrolyse, d'acidogénèse et de méthanogénèse sont séparées dans des volumes adaptables, et qui soit d'une conception simple, robuste, économique et d'une bonne efficacité.

Suivant une caractéristique de l'invention, il est prévu un tel appareil constitué par une enceinte de structure allongée et horizontale, contenant un mélange d'effluents déjà traités et de nouveaux déchets en cours de traitement, les nouveaux déchets à traiter étant introduit à une extrémité de la structure et les effluents traités étant soutirés à l'autre extrémité, ladite enceinte étant divisée en trois compartiments par deux cloisons séparatrices transversales, chaque cloison séparatrice comportant une ouverture à une hauteur légèrement inférieure au niveau de la surface libre du mélange de manière à isoler, d'une part, des deux côtés de sa partie basse, en amont, une masse de mélange contenant une population de bactéries en majorité différentes de celle de la masse située en aval de ladite cloison séparatrice, et, d'autre part, dans sa partie haute, des volumes de gaz de natures différentes surmontant la surface libre de liquide, la première cloison séparatrice séparant ainsi le compartiment de la phase de liquéfaction hydrolytique du compartiment de la phase acidogène, et la seconde cloison séparant le compartiment de la phase acidogène du compartiment de la phase méthanogène, les gaz existants dans chacun des compartiments étant prélévés par des canalisations séparées.

Suivant une autre caractéristique, dans chaque compartiment, est prévu un lit bactérien qui permet de réguler le pH dans le compartiment correspondant.

Suivant une autre caractéristique, le matériau de remplissage du lit bactérien du compartiment méthanogène est constitué par des feuillards de matière plastique tordues en hélices, dont les pas sont choisis dans une pluralité de pas possibles afin d'éviter les imbrications régulières, les hélices pouvant être liées en fagots ou disposées dans une résille pour faciliter la manutention et le décolmatage éventuel.

Suivant une autre caractéristique, dans le compartiment de phase acidogène, sont prévus des moyens pour réinjecter, à une température appropriée, des boues provenant du compartiment de phase méthanogène.

Suivant une autre caractéristique, dans le compartiment de phase méthanogène, sont prévus des moyens pour réinjecter des gaz provenant du compartiment de phase acidogène.

Suivant une autre caractéristique, une cloison transversale déviatrice vers le bas est disposée immédiatement en aval de la première cloison séparatrice, dans le compartiment de phase acidogène, ladite cloison déviatrice dépassant au-dessus du niveau de surface libre et comportant une ouverture dans sa partie basse, un dispositif de dégrillage étant disposé entre la première grille séparatrice et la grille déviatrice.

Suivant une autre caractéristique, la section transversale uniforme de l'enceinte a la forme d'une tranchée en U fermée, à sa partie supérieure, par un couvercle dont les bords sont munis d'une jupe qui plonge au-dessous du fil d'eau.

Suivant une autre caractéristique, le couvercle est oblique.

Suivant une autre caractéristique, au-dessus de l'enceinte, sont installés des caillebotis de stalles de porcherie, et, entre les caillebotis et le haut de l'enceinte, sont installés des moyens de raclage entraînant le lisier vers l'entrée de l'enceinte.

Suivant une autre caractéristique, l'enceinte et son couvercle sont constitués de sections transversales modulaires.

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels:

la Fig. 1 est une vue schématique en perspective d'un appareil suivant l'invention,

la Fig. 2 est une vue en coupe verticale longitudinale de la partie amont de l'appareil de la Fig. 1,

la Fig. 3 est une vue en coupe verticale longitudinale de la partie aval de l'appareil de la Fig. 1,

la Fig. 4 est un schéma indiquant comment positionner les Figs. 2 et 3 pour voir la coupe de l'appareil entier,

la Fig. 5 est une vue en coupe verticale transversale de l'appareil, suivant la ligne V-V de la Fig. 3,

la Fig. 6 est une vue en coupe partielle verticale illustrant, à plus grande échelle, la partie haute de la coupe de la Fig.5,

la Fig. 7 est une vue en plan d'un composant de matériau de remplissage de lit bactérien utilisé dans l'appareil suivant l'invention,

la Fig. 8 est une vue en coupe transversale du composant de la Fig. 7,

la Fig. 9 est une vue en coupe illustrant le montage du broyeur placé à l'entrée de l'appareil suivant l'invention, et

la Fig. 10 est une vue schématique en plan du broyeur de la Fig. 9.

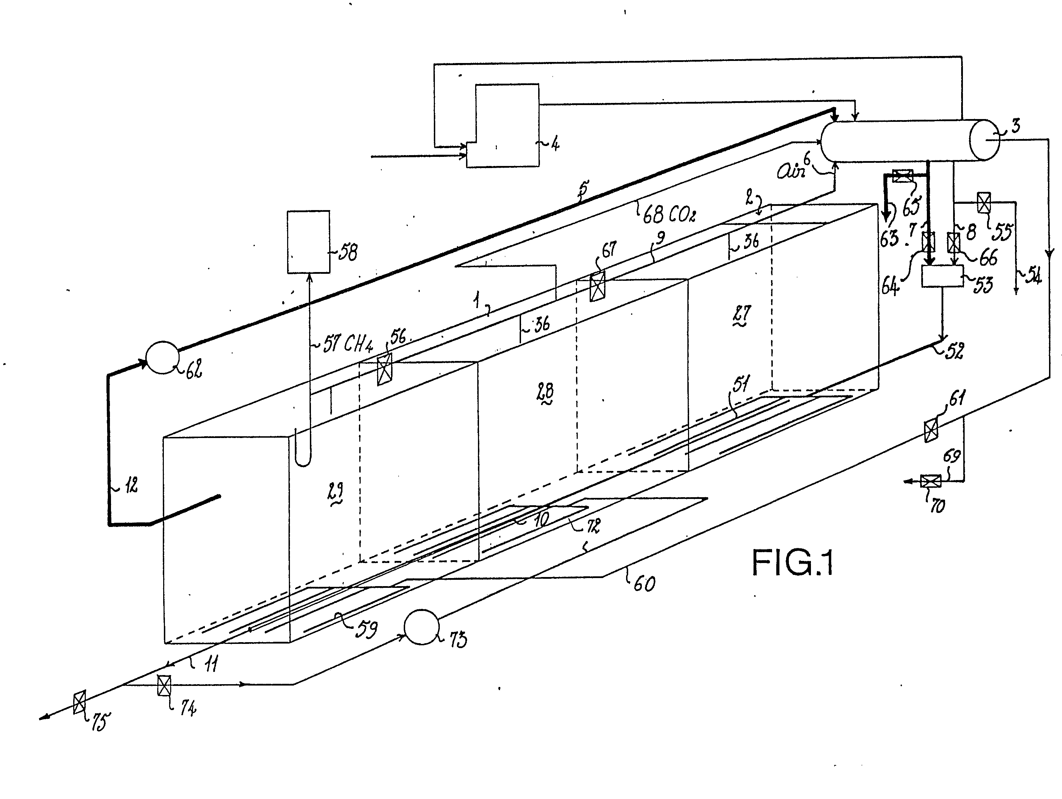

L'appareil de la Fig. 1 comprend une enceinte 1 de forme quasi-parallélipipèdique allongée dans laquelle les déchets biologiques sont introduits à l'entrée 2. L'appareil fait partie d'une installation qui comprend un échangeur de chaleur 3 dont le liquide primaire, qui est généralement de l'eau, est chauffé par une chaudière 4. L'échangeur 3 sert à réchauffer, d'une part, de l'effluent traité qui est introduit par une conduite 5, et, d'autre part, du gaz carbonique, qui est introduit par une conduite 6. L'effluent traité réchauffé sort de l'échangeur 3 par la conduite 7 et le gaz carbonique par la conduite 8. Le long de la partie supérieure de l'enceinte 1, est prévue une conduite de soutirage 9 permettant de soutirer des gaz à l'intérieur de l'enceinte. Le long de la partie basse médiane de l'enceinte est prévue une conduite 10 d'extraction des boues à laquelle est raccordée une conduite 11 de vidange des boues par aspiration. A l'opposé de l'entrée 2, une conduite 12 sert à aspirer l'effluent traité.

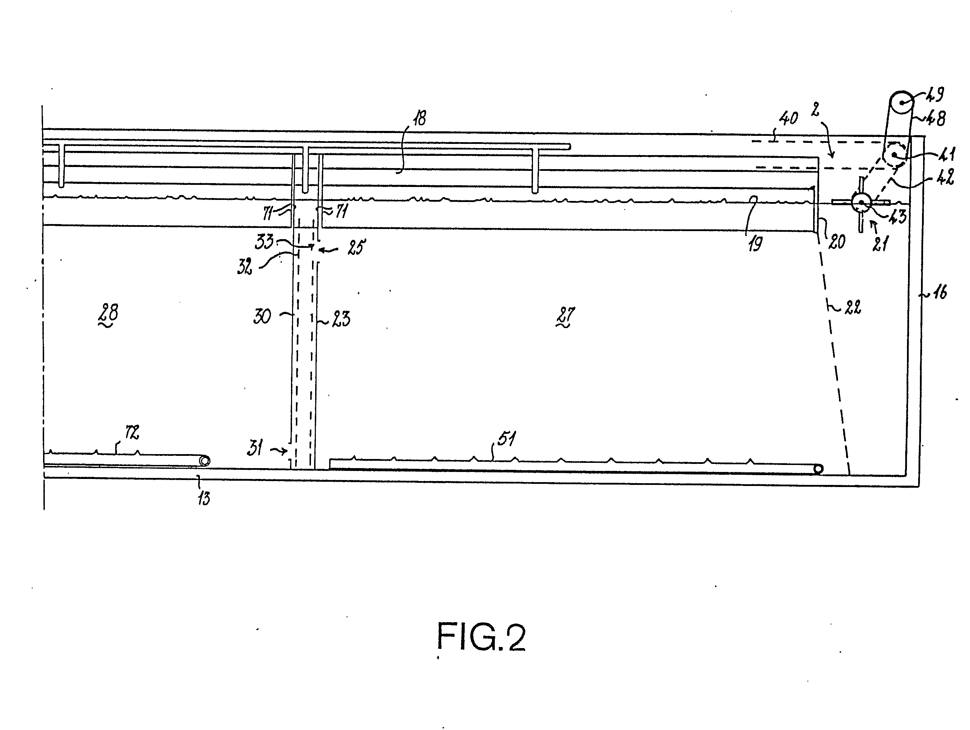

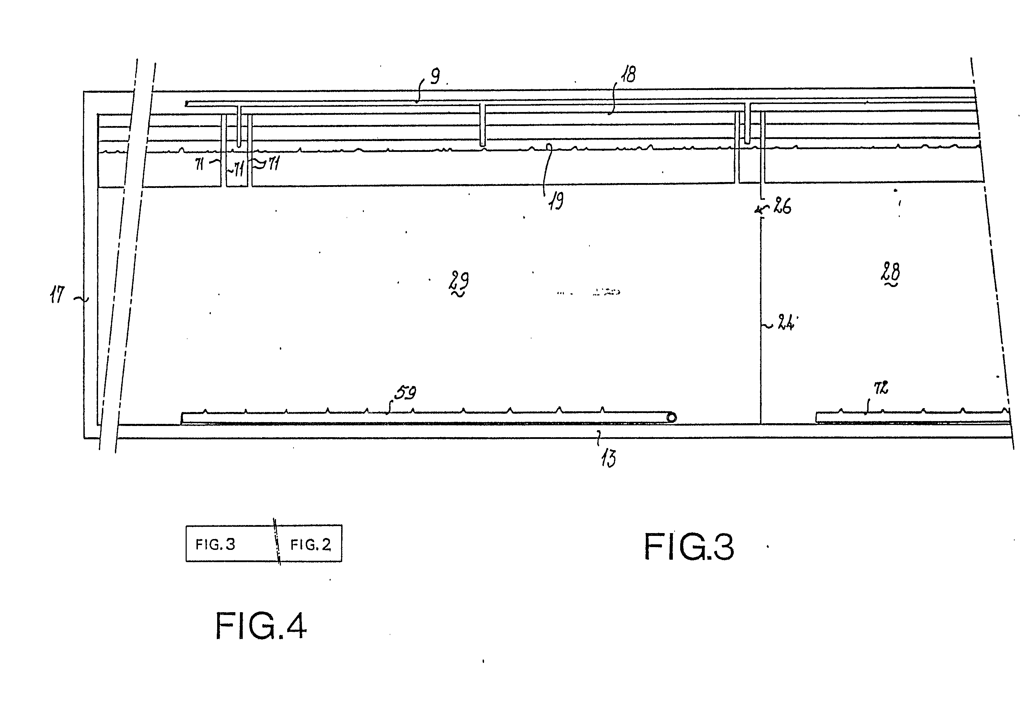

Comme le montrent les Figs. 2, 3 et 5, l'enceinte 1 est formée d'un fond 13, de deux parois longitudinales verticales 14 et 15, de deux parois transversales verticales 16 et 17, et du fond 18 d'un couvercle suspendu aux bords supérieurs des parois 14 et 15. On verra dans la suite comment la structure du couvercle assure l'étanchéité de l'enceinte.

La longueur de l'enceinte 1 est grande par rapport à sa largeur. A titre indicatif, la largeur de l'enceinte 1 peut être de 1,20 m, sa longueur d'une dizaine de mètres et sa hauteur, hors tout, de l'ordre de 2 m.

Le mélange liquide en cours de traitement occupe presque tout le volume de l'enceinte 1 avec une surface libre 19 un peu au-dessous du bord inférieur du fond 18 du couvercle.

A l'entrée 2 de l'appareil, une partie du couvercle est supprimée pour permettre l'introduction du lisier. En pratique, l'entrée 2 a la forme d'une courte cheminée définie par la paroi 16, les parties adjacentes des parois 14 et 15, et une plaque verticale 20 suspendue au bord d'interruption du fond 18 et occupant toute la largeur de l'enceinte. La plaque 20 plonge dans le mélange liquide un peu au-dessous de la surface libre 19. Au niveau de cette surface libre 19, dans l'entrée 2, est installé un broyeur 21 qui sera décrit plus en détail en relation avec les Figs. 9 et 10. Sous le bord inférieur de la plaque 20, est suspendu une grille 22 qui descend jusqu'au fond 13 de l'enceinte.

Il apparaît que la partie de la surface libre 20 en contact avec l'atmosphère est limitée à la section horizontale de l'entrée 2, laquelle est, de plus, partiellement obturée par le broyeur 21, quand celui-ci fonctionne et complètement obturée quand le broyeur est au repos. On a donc bien dans l'enceinte un traitement anaérobie.

En partant de la paroi 16 vers la paroi 17, l'enceinte comporte deux cloisons verticales transversales 23 et 24 formées par des plaques suspendues sous le fond 18 de couvercle et descendant jusqu'au fond 13 de l'enceinte, ces cloisons occupant toute la largeur de l'enceinte. Les cloisons 23 et 24 comportent respectivement deux ouvertures 25 et 26 de section réduite et disposées un peu au-dessous de la surface libre 19. Ces cloisons 23 et 24 divisent donc l'enceinte 1 en trois compartiments 27, 28 et 29 qui sont isolés les uns des autres, sauf les communications restreintes offertes par les ouvertures 25 et 26. En particulier le volume de gaz du compartiment 27 est isolé de l'atmosphère extérieure par la plaque 20 et du volume de gaz du compartiment 28 par le haut de la cloison 23. De même, le volume de gaz du compartiment 28 est isolé de celui du compartiment 29 par le haut de la paroi 24.

En pratique, un peu en aval de la cloison 23, c'est à dire dans le compartiment 28, est suspendue au fond 18 une cloison 30 qui occupe également toute la section droite de l'enceinte et qui présente une ouverture 31 à proximité du fond 13 de l'enceinte. Il en résulte que le flux liquide s'écoulant du compartiment 27 au compartiment 28 doit suivre la chicane créée par les ouvertures 25 et 31. Dans l'intervalle libre entre les cloisons 23 et 30, est monté un panier de dégrillage 32 qui est ouvert en 33 en face de l'ouverture 25 de la cloison 23. Le panier dégrilleur 32 retient les débris solides qui passent par 25, entraînés par le flux liquide. La quantité de ces débris solides est évidemment faible.

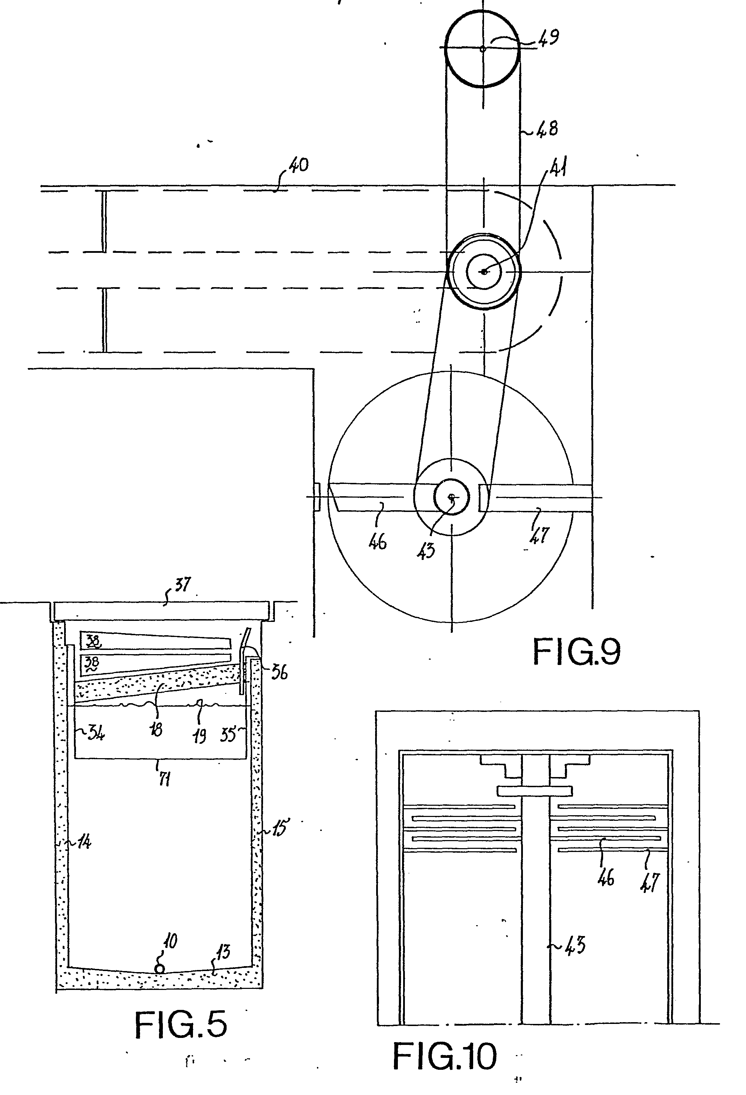

Comme le montre la coupe de la Fig. 5, la surface du fond 13 a la forme d'un dièdre ouvert vers le haut, la conduite 10 étant posée le long de l'arête du dièdre.

Comme le montre la coupe de la Fig. 6, le couvercle de l'enceinte se compose de la plaque de fond 18 qui est fixé, à ses extrémités, à des plaques verticales longitudinales 34 et 35. Les bords supérieurs des plaques 34 et 35 sont repliés vers l'extérieur pour s'appuyer sur les bords supérieurs des parois longitudinales 14 et 15. Par ailleurs, les plaques 34 et 35 descendent au-dessous du niveau de la surface libre 19.

En pratique, la plaque de fond 18 est disposée obliquement, en montant légèrement de la plaque 34 à la plaque 35 (ou vice versa). Il en résulte que l'arête du dièdre formé par 18 et 35 se trouve à un niveau plus élevé que l'arête du dièdre formé par 18 et 34. De place en place, dans la plaque 18, près de l'arête de 18 et 35, est prévu un tronçon de conduite 36 qui est raccordé en dérivation sur la conduite de collecte de gaz 9. La hauteur des plaques 34 et 35, sous la surface libre 19, est choisie en fonction de la pression maximale admissible dans la conduite 9 de manière que la pression de gaz sous le fond 18 ne repousse pas le mélange au-delà du bas des plaques 34 et 35.

Comme le montre particulièrement la coupe de la Fig. 6, l'enceinte 1 peut être directement au-dessous des caillebotis 37 d'une porcherie de manière à ce que les déjections des porcs tombent directement sur la surface supérieure de la plaque 18 entre les hauts des plaques 34 et 35. Un racleur composé de pales transversales 38 montées sur des chaînes sans fin 39 et 40 entraîne le lisier vers l'entrée 2. Comme le montre la Fig. 2, les chaînes 39 et 40 sont entraînées par l'axe 41. Par une transmission à roues dentées et chaîne 42, l'axe 41 entraîne également l'axe 43 du broyeur 21. Pour tenir compte de la position oblique de la plaque de fond 18, les pales 38 sont trapézoïdales. En pratique, les chaînes 39 et 40 sont supportées, au-dessus de 18, par des galets 44 qui roulent dans des chemins de roulement appropriés 45, respectivement fixés sur les plaques 34 et 35.

Comme le montrent les Figs. 9 et 10, le broyeur 21 est un dilacérateur à axe horizontal 43 dont le tambour est constitué par des dents coupantes 46 pénétrant dans des dents fixes 47 fixées, d'une part, à la paroi 16 et, d'autre part, à la plaque 20. La surface libre 19 du mélange, à l'entrée 2, est normalement au niveau des dents 47. La Fig. 9 montre que l'axe 41 est entraîné, par une transmission 48, par l'axe 49 d'un moteur, non montré.

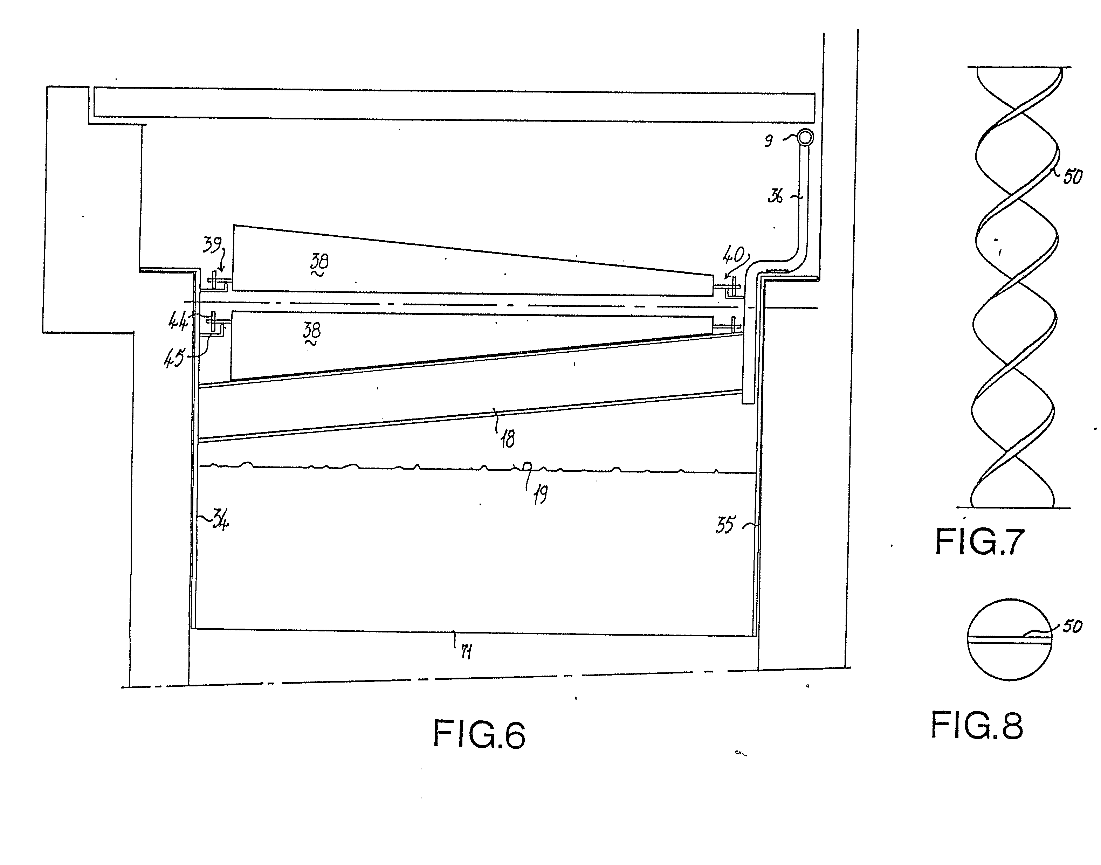

Dans les compartiments 27, 28 et 29, sont disposés des lits bactériens dont le matériau de remplissage est choisi pour obtenir un pH adéquat dans chaque compartiment. Dans le compartiment 29, dans une exemple préféré de réalisation, le lit est constitué par des bottes de rubans rigides torsadés 50 qui sont montrés en plan à la Fig. 7 et en coupe à la Fig. 8. La matière de ces plaques peut être du PVC. Plusieurs pas de torsion sont prévus pour les plaques 50 de manière à ce que, mises en bottes, le tassement soit minimal. Par comparaison avec les matériaux précités, les rubans 50 laisse un volume libre trois fois plus élevé, de l'ordre de 95 % au lieu de 30 %, alors que les surfaces activées disponibles à la population bactérienne ne leur sont inférieures que de 30 %. Les espaces libres, ainsi créés, sont continus et sans étranglement et offrent aux liquides un cheminement préférentiel longitudinal. Le colmatage est facile à annuler en agitant les bottes ainsi constituées.

En se reportant à nouveau à la Fig. 1, il apparaît qu'à la base du compartiment 27, est encore prévu une série de tuyaux perforés 51 qui sont raccordés par une conduite 52 à un raccord 53 dont une entrée est raccordée à la conduite 7, par une vanne 64, et l'autre entrée à la conduite 8, par une vanne 66.

La conduite 8 comporte également une dérivation 54 sur laquelle est prévue une vanne 55 permettant de rejeter du $CO_2$ en excès dans l'atmosphère, ou éventuellement de l'utiliser dans une serre ou un tunnel agricole.

Au-dessus de la cloison 24, est prévue, sur la conduite 9, une vanne d'arrêt 56 qui isole la partie de la conduite 9 courant au-dessus du compartiment 28 de la partie qui court au-dessus du compartiment 29, et une vanne d'arrêt 67 qui sépare la partie de conduite qui court au-dessus de 27 de la partie qui court au-dessus de 28. Comme on le verra dans la suite, la partie de 9 qui se trouve au-dessus de 27 recueille un mélange d'air et de gaz carbonique, celle qui se trouve au-dessus de 28 en majorité du gaz carbonique tandis que la dernière partie de 9 recueille du méthane. La partie de 9 en-deça de 67 est raccordée à la conduite 6. La partie de 9 comprise entre 56 et 67 est raccordée à une conduite 68 qui passe dans l'échangeur 3 et porte, à la sortie de ce dernier, une vanne 61 et une dérivation 69 portant une vanne 70. La partie méthane de 9 est raccordée par une conduite 57 à un gazomètre 58.

A la base du compartiment 29, est encore prévu une série de tuyaux perforés 59 qui sont reliés par une conduite 60 à la sortie de la vanne 61.

La conduite 12 d'aspiration de l'effluent liquide traité est reliée, par l'intermédiaire d'une pompe 62, à la conduite 5, elle-même reliée à la conduite 7. En pratique, la conduite 7 comporte une dérivation 63 permettant le rejet de tout ou partie des effluents traités. Sur la conduite 7 et sur la dérivation 63, sont encore prévues des vannes 64 et 65 de réglage de débit permettant de régler la part d'effluent traité à recycler et la part des effluents traités rejetés.

Le fonctionnement de l'installation qui vient d'être décrite va maintenant être décrit.

Le compartiment 27 est le siège de la phase d'hydrolyse liquéfaction des matières broyées par le broyeur 21 et entrant dans l'appareil. Les effluents déjà traités, réchauffés et renvoyés dans le compartiment 27 par les tuyaux 51 servent de solvant et subissent un nouveau traitement. Le gaz carbonique également réinjecté par les tuyaux 51 sert à l'agitation mécanique du mélange. La flore bactérienne comporte en majorité des bactéries provoquant l'hydrolyse.

Donc, le mélange qui passe par l'ouverture 25 ne comporte pratiquement plus de matières solides. Au-dessus de la surface libre 19 du compartiment 27, le gaz carbonique est repris par la conduite 9, à travers les tronçons 36 correspondant. Les rares matières solides entraînées à travers 25 sont retenues dans le panier 32.

Le compartiment 28 est le siège de la phase acidogène. La flore bactérienne comporte une majorité de bactéries transformant des composants des effluents en acides organiques, avec une majorité d'acides acétique et carbonique. Au-dessus de la surface libre du mélange, se dégage du gaz carbonique recueilli par la partie correspondante de la conduite 9. Une partie des boues sortant par la conduite 11 est réinjectée dans le compartiment 28 par des tuyaux 72, par l'intermédiaire d'une pompe 73 et d'une vanne 74. Une vanne 75 commande le rejet des boues à l'extérieur à partir de 11. On peut également prévoir un apport de sels minéraux pour accélérer les réactions.

Le compartiment 29 est le siège de la phase méthanogène. Le lit bactérien comporte une majorité de bactéries provoquant la méthanisation des acides provenant du compartiment 28 par l'ouverture 26. Le gaz carbonique injecté par les tuyaux 59 accélèrent ces réactions.

En pratique, les ségrégations des bactéries dans les trois compartiments se font naturellement à la condition de choisir correctement les volumes des compartiments 27 à 29, c'est à dire leurs longueurs respectives, en fonction du volume et des caractéristiques de l'effluent à traiter. Il faut bien entendu que les températures d'action des bactéries soient respectées ce qui est obtenu en calorifugeant le fond 13, le couvercle 18 et les parois 14 à 17, spécialement en ce qui concerne le compartiment 29, et en régulant la température de l'échangeur de chaleur 3 de manière à avoir par exemple constamment 37° C dans le compartiment 27. Le fond 18 du couvercle nécessite un calorifugage soigneux, bien que le lisier dont la température est supérieure à celle qui règne dans la porcherie apporte des calories utiles.

Pour pouvoir ajuster facilement la longueur des compartiments 27 à 29 à la quantité de lisier à traiter, on prévoit des éléments modulaires pour constituer les parois 16, 15 et le fond 13, ces éléments étant mis bout à bout et complétés par des éléments d'extrémi-

tés, l'un comportant l'entrée 2 et la paroi 16, et l'autre la paroi 17. De même, le couvercle est formé d'éléments modulaires. En ce qui concerne les cloisons 23 et 24, on peut prévoir de les monter directement sur des éléments de couvercle plus courts que les éléments modulaires courants de couvercle et d'associer à ces éléments courts de couvercle, des éléments de parois et de fond de même longueur. Sous l'élément de couvercle portant la cloison 23, est également accrochée la cloison 30 et le panier 32. En relevant cet élément de couvercle, au cours du fonctionnement de l'installation, on peut procéder au vidage du panier. Par ailleurs, si l'on désire modifier allonger un compartiment en raccourcissant le voisin, on retire l'élément court de couvercle correspondant et on décale un ou plusieurs éléments courants pour le replacer à la distance voulue.

En pratique, les éléments de couvercle comporte sous le fond 18, non seulement les plaques 34 et 35, mais également des plaques transversales 71 de même hauteur pour isoler le gaz, et chaque élément est pourvu d'un tronçon 36.

Le débit global de l'appareil est évidemment déterminé par le volume des nouveaux éffluents à traiter. Le flux d'écoulement dans l'appareil dépend également de cette valeur, mais également du débit de la pompe 62. Cette pompe peut fonctionner en continu ou en discontinu, et en fonction de la fréquence d'arrivée des nouveaux effluents.

## REVENDICATIONS

1) Appareil de digestion anaérobie de déchets biologiques dans lequel les phases d'hydrolyse liquéfactrice, acidogène et méthanogène sont séparées, caractérisé en ce qu'il est constitué par une enceinte (1) de structure allongée et horizontale, contenant un mélange d'effluents déjà traités et de nouveaux déchets en cours de traitement, les nouveaux déchets à traiter étant introduit à une extrémité de la structure et les effluents traités étant soutirés à l'autre extrémité, ladite enceinte étant divisée en trois compartiments (27, 28, 29) par deux cloisons séparatrices transversales (25, 26), chaque cloison séparatrice comportant une ouverture à une hauteur légèrement inférieure au niveau de la surface libre du mélange de manière à isoler, d'une part, des deux côtés de sa partie basse, en amont, une masse de mélange contenant une population de bactéries en majorité différentes de celle de la masse située en aval de la dite cloison séparatrice, et, d'autre part, dans sa partie haute, des volumes de gaz de natures différentes surmontant la surface libre de liquide, la première cloison séparatrice séparant ainsi le compartiment de la phase d'hydrolyse liquéfactrice (27) du compartiment de la phase acidogène (28), et la seconde cloison séparant le compartiment de la phase acidogène (28) du compartiment de la phase méthanogène (29), les gaz existants dans chacun des compartiments étant prélévés par des canalisations séparées (6, 68, 57).

2) Appareil suivant la revendication 1, caractérisé en ce que, dans chaque compartiment, est prévu un lit bactérien régulant le pH du compartiment considéré.

3) Appareil suivant la revendication 2, caractérisé en ce que le matériau de remplissage du lit bactérien du compartiment de la phase méthanogène (29) est constitué par des feuillards (50) de matière plastique tordues en hélices, dont les pas sont choisis dans une pluralité de pas possibles afin d'éviter les imbrications régulières.

4) Appareil suivant l'une des revendications 1 à 3, caractérisé en ce que, dans le compartiment de phase acidogène (28), sont prévus des moyens (72) pour réinjecter des boues provenant du compartiment de phase méthanogène (29), avec éventuellement un apport de sels minéraux.

13

5) Appareil suivant l'une des revendications 1 à 4, caractérisé en ce que, dans le compartiment de phase d'hydrolyse (27), sont prévus des moyens (51) pour réinjecter de l'air et de l'effluent provenant du compartiment de phase méthanogène (29).

6) Appareil suivant l'une des revendications 1 à 5, caractérisé en ce que, dans le compartiment de phase méthanogène (29), sont prévus des moyens (59) pour réinjecter du gaz carbonique.

7) Appareil suivant l'une des revendications 1 à 6, caractérisé en ce qu'une cloison transversale déviatrice (30) est disposée en aval de la première cloison séparatrice (23), dans le compartiment de phase acidogène (28), ladite cloison déviatrice dépassant au-dessus du niveau de surface libre et comportant une ouverture (31) dans sa partie basse, un dispositif de dégrillage (32) étant disposé entre la première grille séparatrice (23) et la grille déviatrice (30).

8) Appareil suivant l'une des revendications 1 à 7, caractérisé en ce que la section transversale uniforme de l'enceinte a la forme d'un U fermé, à sa partie supérieure, par un couvercle (18) dont les extrémitées sont légèrement au-dessous des bouts des branches du U.

9) Appareil suivant la revendication 8, caractérisé en ce que ledit couvercle (18) est oblique.

10) Appareil suivant l'une des revendications 1 à 9, caractérisé en ce qu'au-dessus de l'enceinte, sont installés des caillebotis (37) de stalles de porcherie, et, entre les caillebotis et le haut de l'enceinte, sont installés des moyens de raclage (38) entraînant le lisier vers l'entrée de l'enceinte.

11) Appareil suivant l'une des revendications 1 à 10, caractérisé en ce que l'enceinte est constituée de sections transversales modulaires permettant de donner aux différents compartiments les longueurs désirées.

FIG.1

FIG.2

FIG.3

| FIG.3 | FIG.2 |

FIG.4

0048675

FIG.9

FIG.5

FIG.10

FIG.6

FIG.7

FIG.8

0048675

0048675

Office européen
des brevets

**RAPPORT DE RECHERCHE EUROPEENNE**

Numéro de la demande

EP 81 40 1470

| | **DOCUMENTS CONSIDERES COMME PERTINENTS** | | | CLASSEMENT DE LA DEMANDE (Int. Cl.³) |
|---|---|---|---|---|
| Catégorie | Citation du document avec indication, en cas de besoin, des parties pertinentes | Revendication concernée | | |
| A | FR - A - 2 324 581 (HITACHI) | | | C 02 F 3/28<br>A 01 C 3/02 |
| A | US - A - 4 022 665 (S. GHOSH et al.) | | | |
| A | US - A - 4 100 023 (B.A. McDONALD) | | | |
| A | CH - A - 436 147 (G. DUNAND et al.) | | | |
| A | FR - A - 773 836 (H. VAUBOURG) | | | |
| | ---- | | | |

DOMAINES TECHNIQUES RECHERCHES (Int. Cl.³)

C 02 F 3/28
C 12 M 1/00
A 01 C 3/02

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 27-11-1981 | TEPLY |

OEB Form 1503.1  06.78